# DEMANDE DE BREVET EUROPEEN

(11) **EP 2 893 909 A2**
(43) Date de publication de la demande: **15.07.2015**
(21) Numéro de dépôt: 14199398.0
(22) Date de dépôt: 19.12.2014
(51) Int. Cl.: A61F 5/08, A62B 23/06

(54) **DISPOSITIF NASAL AMBULATOIRE**

(30) Priorité: 03.01.2014 FR 1450023
(71) Demandeur: Creps, Georges, 78124 Montainville (FR)
(72) Inventeur: Creps, Georges, 78124 Montainville (FR)
(74) Mandataire: Casalonga

(57) **Abrégé**

Ce dispositif nasal ambulatoire d'assistance respiratoire comporte au moins une structure d'expansion intra-narinaire (2) destinée à être insérée dans une narine, la structure d'expansion présentant une forme pyramidale à base (3) annulaire et comportant un arceau (4) s'étendant à partir de ladite base (3).

## Description

La présente invention concerne un dispositif permettant d'améliorer le passage, par voie nasale, de l'air inspiré ou expiré par une personne présentant une configuration anatomique particulière, obstructive des voies narinaires et des cloisons nasales, cette obstruction réduisant la ventilation en l'absence d'une assistance mécanique à l'ouverture des voies respiratoires nasales muco-cartilagineuses.

Cette assistance mécanique est traditionnellement effectuée au niveau des narines par des systèmes d'aide à leur ouverture.

Certains systèmes comportent une ailette venant se positionner dans chaque narine, plaquée sur la muqueuse. Les ailettes sont reliées entre elles par un arceau faisant office d'écarteur des deux ailettes. Un tel arceau externe est cependant disgracieux et peu efficace dans le maintien en place du système.

D'autres systèmes comportent un tube vertical interne destiné à être inséré dans chaque narine. Les tubes sont reliés entre eux par une entretoise sous-nasale externe. Certains de ces systèmes intègrent des réservoirs de substances thérapeutiques.

Il a été constaté que ces systèmes sont inconfortables et disgracieux. En outre, ils n'éliminent pas le risque de délogement ou de dégagement du système, en particulier au cours du sommeil. Par ailleurs, ces systèmes ne permettent pas d'obtenir une répartition anatomique mesurée et élargie des forces d'appui à exercer, de façon homothétique, tant au niveau des zones muco-cartilagineuses des narines qu'au niveau des zones muco-cartilagineuses des voies aériennes nasales supérieures. Enfin, ces systèmes sont susceptibles d'engendrer un blocage sécrétoire physiologique des muqueuses dû au contact intime de leur structure avec la muqueuse nasale.

Il a par ailleurs été proposé d'utiliser des dispositifs ambulatoires fondés sur l'utilisation de ballonnets destinés chacun à être insérés dans une narine et présentant une extrémité dotée d'un orifice et une paroi ajourée.

On pourra à cet égard se référer au document FR 1 200 102 qui décrit un tel dispositif.

Bien qu'avantageux à maints égards, un tel dispositif présente une efficacité limitée dans le respect du flux sécrétoire, même s'il présente cependant un intérêt certain en tant que support hémostatique, respectant le passage de l'air inspiré ou expiré dans un contexte post opératoire nasal.

Le dispositif selon l'invention permet de remédier à ces inconvénients.

L'invention a ainsi pour objet un dispositif nasal ambulatoire d'assistance respiratoire, comprenant au moins une structure d'expansion intra-narinaire destinée à être insérée dans une narine, dans lequel la structure d'expansion présente une forme pyramidale à base annulaire et comporte au moins un arceau s'étendant à partir de ladite base.

Les structures d'expansion sont destinées à être insérées dans un site receveur, en l'espèce le site intra narinaire et les fosses nasales. La base annulaire des structures d'expansion, tournée vers l'extérieur lorsqu'elles sont insérées dans une narine, est ouverte afin de favoriser l'ouverture de la narine.

Il a été constaté qu'un tel dispositif comprenant une structure d'expansion pyramidale comprenant un arceau et une base annulaire respecte la physiologie des muqueuses nasales, en évitant les bloquages des sécrétions dues à l'activité des fonctions physiologiques de la muqueuse nasale.

Selon une caractéristique, le dispositif est au moins en partie réalisé en un matériau élastiquement déformable à mémoire de forme.

Ainsi les structures d'expansion pyramidales, lorsqu'elles sont introduites dans les narines, par déformation compressive, épousent par une extension homothétique, dans la recherche de la forme initiale, les reliefs anatomiques rencontrés. Ces reliefs ont une anatomie propice rétentive des structures d'expansion pyramidales évitant leur délogement en particulier au cours du sommeil.

On notera que le dispositif est avantageusement réalisé par moulage d'une matière plastique et que le degré d'élasticité du dispositif est choisi de manière à obtenir une pression homothétique mesurée sur les tissus muqueux sur lesquels le dispositif s'appuie.

Dans un mode de réalisation, chaque structure d'expansion comporte un arceau principal de forme générale triangulaire, un anneau principal relié à la base de l'arceau principal et au moins une branche reliée à l'arceau principal et audit anneau.

On peut en outre prévoir au moins un anneau intermédiaire relié à l'arceau principal et disposé entre le sommet de l'arceau principal et la base annulaire.

Dans tous les cas, quel que soit le nombre d'arceaux et d'anneaux, une telle structure est naturellement adaptée au respect des régions anatomiques sur lesquelles elle s'appuie, en respectant la physiologie des autres régions anatomiques.

Avantageusement, pour certaines applications thérapeutiques, le sommet de la structure d'expansion comporte un écarteur d'extrémité comprenant au moins un appendice digitalé destiné à venir se positionner dans la région du cartilage triangulaire ou de manière générale dans des régions anatomiques éloignées des fosses nasales.

Comme le reste de la structure d'expansion pyramidale, les appendices digitalés procurent un appui sélectif sur la muqueuse pour éviter le blocage des sécrétions. En d'autres termes, les appuis restent localisés tout en procurant une assistance mécanique apte à maintenir ouvertes les voies respiratoires nasales muco-cartilagineuses.

On peut par ailleurs prévoir que le dispositif comporte au moins un réservoir interne.

De tels réservoirs peuvent présenter diverses formes, par exemple tubulaire. Ils peuvent être fixés au coeur des structures d'expansion et sont notamment destinés à recevoir des substances thérapeutiques diffusantes pour diverses applications cliniques.

On notera à cet égard que la constitution des structures d'expansion qui comportent notamment un arceau et une base annulaire est particulièrement adaptée pour laisser libre les surfaces des muqueuses qui bénéficient dès lors des substances diffusées.

Dans un mode de mise en oeuvre, le dispositif comporte une paire de structures d'expansion intra-narinaire reliées par une entretoise.

On pourra à cet égard utiliser une entretoise stabilisante, peu visible, confortable et transparente. Cette entretoise est destinée à venir se placer sous la cloison nasale externe et est notamment destinée à faciliter l'insertion et le retrait du dispositif.

Selon une autre caractéristique du dispositif, chaque structure d'expansion intra-narinaire comporte une paroi transversale située sur le trajet du flux aérien.

Par exemple, la paroi est pleine ou ajourée.

Avantageusement, la structure d'expansion intra-narinaire est réalisée à partir d'un matériau perspirant.

Par exemple, le matériau perspirant est résorbable.

D'autres buts, caractéristiques et avantages apparaîtront à la lecture de la description suivante, donnée uniquement à tant qu'exemple non limitatif, et faite en référence aux dessins annexés sur lesquels :
- la figure 1 est une vue générale d'un dispositif nasal ambulatoire selon un mode de réalisation de l'invention ;
- la figure 2 est une vue générale de profil du dispositif nasal ambulatoire selon un mode de réalisation de l'invention, placé dans une narine ;
- la figure 3 montre une vue de face du dispositif de la figure 2, installé dans une narine gauche ;
- la figure 4 représente le trajet de l'air inspiré du cartilage alaire vers le cartilage triangulaire puis vers les fosses nasales ; et
- la figure 5 montre une vue de face d'un autre exemple de mise en oeuvre d'un dispositif selon l'invention, installé dans une narine.

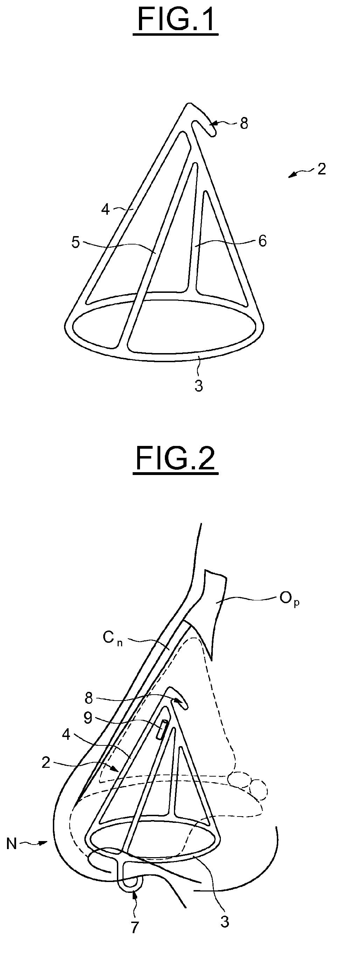

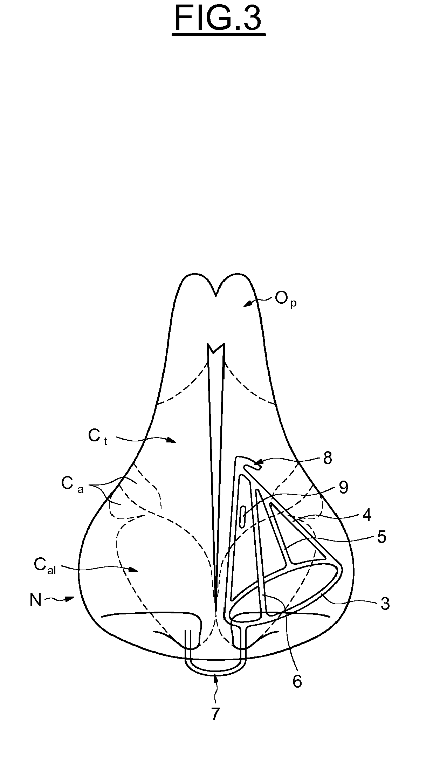

On se référera tout d'abord à la figure 1 qui illustre la structure de base d'un dispositif nasal ambulatoire conforme à l'invention et aux figures 2 et 3 qui montrent un exemple de mise en oeuvre d'un dispositif nasal ambulatoire inséré dans une narine N d'un utilisateur.

Comme on le voit, le dispositif comporte, dans l'exemple de réalisation représenté sur ces figures, deux structures d'expansion intra-narinaire 2 destinées chacune à être insérées dans une narine d'un utilisateur.

La dimension des structures d'expansion est un standard anatomique et doit répondre à deux tailles : « large » et « médium ».

Chaque structure d'expansion intra-narinaire a une forme globalement pyramidale et comporte une base annulaire 3, c'est à dire une base ouverte, et un ou plusieurs arceaux 4 de forme générale triangulaire s'étendant à partir de la base annulaire.

La structure d'expansion intra-narinaire comporte en outre une ou deux branches latérales 5 et 6 dont les extrémités sont reliées à l'arceau 4 et à la base annulaire 3. Les branches sont ici réunies au voisinage de leur extrémité par laquelle elles sont reliées à l'arceau 4 de sorte que ces branches forment un arceau secondaire.

Les structures d'expansion intra-narinaire sont reliées par une entretoise 7 plate, incurvée et discrète à la base du nez.

Comme indiqué précédemment, et en se référant aux figures 2 et 3 sur lesquelles on reconnaît les os propres du nez Op, la cloison nasale Cn, le cartilage triangulaire Ct, les cartilages accessoires Ca et le cartilage alaire Cal, les structures d'expansion intra-narinaire sont destinées à être insérées dans les narines d'un utilisateur. La surface de la base 3 de chaque structure d'expansion est supérieure à la lumière de la narine pour assurer leur maintien in situ. Les structures d'expansion intra-narinaire exercent en réalité un appui sur la muqueuse nasale sur toute la longueur de la base annulaire, de l'arceau 4 et des branches latérales 5 et 6 pour favoriser l'ouverture de la narine, tout en évitant le blocage des sécrétions.

Les structures d'expansion peuvent par ailleurs être pourvues d'une ou plusieurs extensions digitalées de forme variable, planes ou incurvées, judicieusement placées pour exercer une pression muco-cartilagineuse ciblée. Les dimensions des extensions sont relatives à l'anatomie qu'elles rencontrent.

A cet égard, les structures d'expansion sont ici dotées d'un écarteur d'extrémité 8 prévu au sommet de l'arceau 4 et comprenant au moins un appendice digitalé réalisé sous la forme d'un doigt recourbé destiné à venir se positionner et à exercer un appui sur la région du cartilage triangulaire pour venir l'écarter.

Un tel écarteur reste toutefois optionnel et pourrait dès lors être omis.

Dans le mode de réalisation des figures 2 et 3, chaque structure d'expansion peut en outre être dotée intérieurement d'un réservoir 9 destiné à recevoir une substance thérapeutique. Un tel réservoir peut par exemple être fixé du côté intérieur de la structure d'expansion, par exemple sur l'arceau ou sur les branches latérales de manière à être situé sur le trajet du flux d'air tout en restant à distance des muqueuses. Un tel réservoir peut avoir une inclinaison, un emplacement ou une forme différente selon sa fonction, par exemple diffuseur, thérapies diverses, etc ....

L'ensemble des éléments est réalisable en une seule opération de moulage par injection, ce qui donne une parfaite homogénéité à l'ensemble de la structure. On réalisera de préférence le dispositif par moulage par injection d'un matériau à mémoire de forme apte à exercer des efforts sur les zones muco-cartilagineuses sur lesquelles il s'appuie et présentant des propriétés physiques, notamment une élasticité, choisies de manière à régler l'effort qu'il exerce sur ces zones.

On notera par ailleurs que, comme indiqué précédemment, chaque structure d'expansion a une structure élastiquement déformable notamment grâce à sa réalisation sous la forme d'une structure pyramidale comprenant une base annulaire et un arceau s'étendant à partir de la base annulaire.

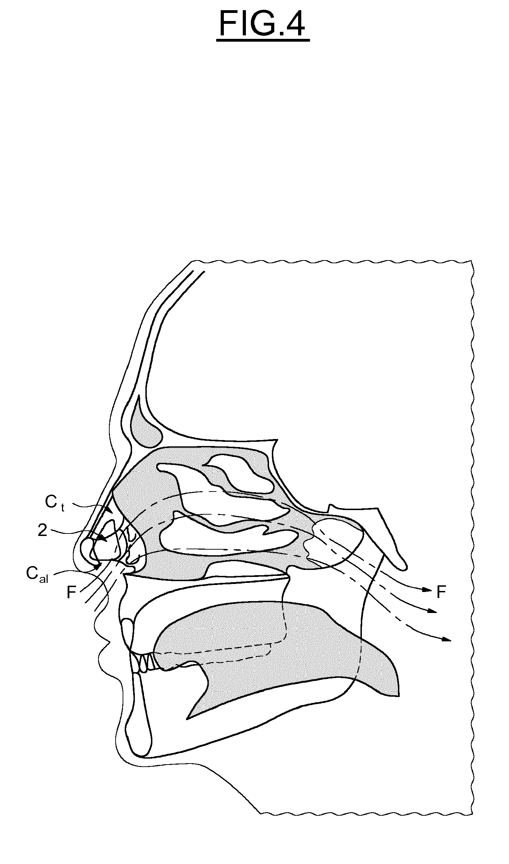

En outre, comme visible par les flèches F sur la figure 4, l'air aspiré à travers la base annulaire 3 circule jusqu'à l'extrémité opposée à travers la paroi ajourée de la structure d'expansion pour suivre son trajet vers les fosses nasales.

Le dispositif selon l'invention est particulièrement destiné à l'industrie médico-chirurgicale et la thérapie concernant la sphère Oto Rhyno Laryngologique.

Dans diverses applications, il peut être utilisé pour assurer une fonction d'écarteur et présenter des orientations particulières adaptées à l'espace rencontré, selon l'organe à rectifier, afin de procurer une solution efficace à la gêne respiratoire ressentie par un utilisateur, résultant d'un obstacle architectural ou muqueux dans le trajet des flux aériens.

Ainsi, le dispositif nasal ambulatoire procure en premier lieu une assistance respiratoire grâce à la présence des structures d'expansion s'insérant dans les narines et facilitant la ventilation grâce à l'ouverture des voies respiratoires améliorant le volume d'air inspiré ou expiré.

Un tel dispositif permet également de procurer un certain nombre d'effets thérapeutiques.

En premier lieu, la structure d'expansion assure une fonction de guidage osseux, cartilagineux ou muqueux dans la mesure où, par exemple, le maintien d'un cartilage dans une position, sous l'action d'un effort exercé par la structure, peut avoir un effet durable.

On notera néanmoins que, outre les effets thérapeutiques, grâce à sa fonction de guidage osseux, cartilagineux ou muqueux, la structure d'expansion peut également être utilisée à des fins esthétiques.

Comme indiqué précédemment, la structure d'expansion peut également agir en tant qu'écarteur au niveau d'obstacles anatomiques vers les fosses nasales.

Lorsque la fonction de guidage ou d'écarteur est recherchée, le dispositif nasal permet d'obtenir une stabilité durable du site muco-cartilagineux dans lequel il s'insère, et est particulièrement efficace pour la réduction de défauts anatomiques architecturaux ou muqueux de la valve nasale et de la cloison nasale.

Il a été constaté qu'un tel dispositif est parfaitement efficace pour s'adapter et solliciter à l'écartement le cartilage triangulaire et le cartilage alaire en cas de déficience de ces derniers.

Le dispositif selon l'invention peut ainsi trouver une application particulièrement intéressante pour corriger les anomalies du cartilage triangulaire responsables d'une perte de l'intégrité de la valve nasale et d'une perturbation consécutive du flux ventilatoire et lui permettre de retrouver sa fonction d'origine, en venant s'insérer notamment entre le cartilage triangulaire et le cartilage alaire.

Ainsi, le dispositif nasal ambulatoire selon l'invention a en premier lieu pour objectif de fournir une assistance respiratoire à un utilisateur. Il peut bien entendu s'agir d'un usage privé pour procurer une assistance respiratoire autonome nocturne à des utilisateurs.

Il peut toutefois également s'agir de procurer une assistance respiratoire post-opératoire, en particulier au réveil d'un patient, lorsque les valves respiratoires l'empêchent de respirer.

Par ailleurs, grâce à sa fonction de guidage osseux, cartilagineux ou muqueux et d'écartement d'obstacles anatomiques vers les fosses nasales, le dispositif peut fournir une alternative à des solutions chirurgicales dédiées au traitement de pathologies obstructives réduisant la ventilation, en fournissant une solution orthopédique capable de traiter durablement une configuration anatomique obstructive des voies narinaires et/ou des cloisons nasales.

En second lieu, comme indiqué précédemment, l'intérieur de la structure d'expansion intra-narinaire peut présenter un réservoir permettant la diffusion de substances thérapeutiques efficaces pour le traitement de pathologies d'obstruction nasale et plus généralement de la conduction de l'air dans les voies aériennes supérieures. Tel peut être en particulier le cas pour la diffusion in situ de substances thérapeutiques agissant sur des problèmes de gonflement de volets muqueux, par exemple des cornets.

Il peut également s'agir de fournir un accompagnement à la cicatrisation dans le cadre d'une chirurgie plastique nasale ou d'une assistance à l'hémostase.

Par ailleurs, grâce à la présence de réservoirs diffuseurs de substances thérapeutiques, un tel dispositif nasal peut trouver application à l'aide au sevrage tabagique ou à tout autre type d'application nécessitant la diffusion d'une substance thérapeutique par voie nasale.

On notera enfin que l'invention n'est pas limitée au mode de réalisation décrit en référence aux figures 1 à 3.

En effet, dans le mode de réalisation décrit précédemment, la structure d'expansion intra-narinaire comporte un seul anneau formant la base de la structure pyramidale et un arceau s'étendant à partir de l'anneau.

Il serait bien entendu possible, en variante de doter la structure d'expansion intra-narinaire d'un ou de plusieurs anneaux intermédiaires reliés à l'arceau principal et disposés par exemple entre le sommet de l'arceau principal et la base annulaire.

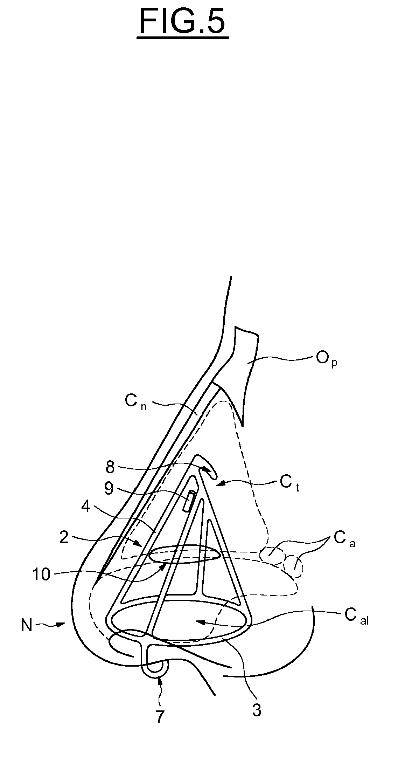

De même, en se référant à la figure 5, chaque structure d'expansion intra-narinaire peut être dotée d'une cloison transversale 10, schématiquement représentée.

Une telle cloison peut, par exemple, être constituée par une paroi pleine afin, dans ce cas, de constituer un obturateur de narines utilisable, par exemple, par des nageurs.

Une telle cloison 10 peut également adopter une structure ajourée de manière à constituer un filtre de sorte que le dispositif peut avantageusement être utilisé dans un environnement pollué.

## Revendications

1. Dispositif nasal ambulatoire d'assistance respiratoire, comprenant au moins une structure (2) d'expansion intra-narinaire destinée à être insérée dans une narine, **caractérisé en ce que** la structure d'expansion présente une forme pyramidale à base (3) annulaire et comporte au moins un arceau s'étendant à partir de ladite base.

2. Dispositif selon la revendication 1, **caractérisé en ce qu'**il est au moins en partie réalisé en un matériau élastiquement déformable à mémoire de forme.

3. Dispositif selon l'une des revendications 1 et 2, **caractérisé en ce que** chaque structure (2) d'expansion comporte un arceau principal (4) de forme générale triangulaire, un anneau principal (3) relié à la base de l'arceau principal et au moins une branche (5, 6) reliée à l'arceau principal et audit anneau.

4. Dispositif selon la revendication 3, **caractérisé en ce que** chaque structure d'expansion comporte au moins un anneau intermédiaire relié à l'arceau principal et disposé entre le sommet de l'arceau principal et la base annulaire.

5. Dispositif selon l'une quelconque des revendications 1 à 4, **caractérisé en ce que** le sommet de la structure d'expansion comporte un écarteur d'extrémité comprenant au moins un appendice digitalé (8) destiné à venir se positionner dans la région du cartilage triangulaire.

6. Dispositif selon l'une quelconque des revendications 1 à 5, **caractérisé en ce qu'**il comporte au moins un réservoir interne (9).

7. Dispositif selon l'une quelconque des revendications 1 à 6, **caractérisé en ce qu'**il comporte une paire de structures d'expansion intra-narinaire reliées par une entretoise (7).

8. Dispositif selon l'une quelconque des revendications 1 à 7, **caractérisé en ce que** chaque structure d'expansion intra-narinaire comporte une paroi transversale (10) située sur le trajet du flux aérien.

9. Dispositif selon la revendication 8, **caractérisé en ce que** la paroi (10) est pleine ou ajourée.

10. Dispositif selon l'une quelconque des revendications 1 à 9, **caractérisé en ce que** la structure d'expansion intra-narinaire est réalisée à partir d'un matériau perspirant.

11. Dispositif selon la revendication 10, **caractérisé en ce que** le matériau perspirant est résorbable.
